# EUROPÄISCHE PATENTSCHRIFT

(11) **EP 0 868 527 B1**
(45) Veröffentlichungstag und Bekanntmachung des Hinweises auf die Patenterteilung: **10.01.2007**
(21) Anmeldenummer: 96946220.9
(22) Anmeldetag: 18.12.1996
(51) Int. Cl.: C12P 13/08, C07K 14/34

(54) **VERFAHREN ZUR MIKROBIELLEN HERSTELLUNG VON AMINOSÄUREN DURCH GESTEIGERTE AKTIVITÄT VON EXPORTCARRIERN**
PROCESS FOR THE MICROBIAL PRODUCTION OF AMINO ACIDS BY BOOSTED ACTIVITY OF EXPORT CARRIERS
PROCEDE DE PREPARATION MICROBIENNE D'AMINOACIDES PAR ACTIVITE ACCRUE DE VECTEURS DE TRANSPORT

(30) Priorität: 22.12.1995 DE 19548222
(43) Veröffentlichungstag der Anmeldung: 07.10.1998
(73) Patentinhaber: Forschungszentrum Jülich GmbH, 52425 Jülich (DE)
(72) Erfinder: VRLIJC, Marina, D-52428 Jülich (DE); EGGELING, Lothar, D-52428 Jülich (DE); SAHM, Hermann, D-52428 Jülich (DE)
(74) Vertreter: Münch, Volker
(86) Internationale Anmeldenummer: PCT/DE1996/002485
(87) Internationale Veröffentlichungsnummer: WO 1997/023597

(56) Entgegenhaltungen:
- WO-A-95/19442
- MOL MICROBIOL, DEC 1996, 22 (5) P815-26, ENGLAND, XP000675494 VRLJIC M ET AL: "A new type of transporter with a new type of cellular function: L-lysine export from Corynebacterium glutamicum."
- JOURNAL OF BACTERIOLOGY, Bd. 177, Nr. 20, Oktober 1995, Seiten 5991-5993, XP000608713 WEHRMANN A ET AL: "FUNCTIONAL ANALYSIS OF SEQUENCES ADJACENT TO DAPE OF CORYNEBACTERIUM GLUTAMICUM REVEALS THE PRESENCE OF AROP, WHICH ENCODES THE AROMATIC AMINO ACID TRANSPORTER"
- JOURNAL OF FERMENTATION AND BIOENGINEERING, Bd. 78, Nr. 6, 1994, Seiten 420-425, XP000608032 IKEDA M ET AL: "TRANSPORT OF AROMATIC AMINO ACIDS AND ITS INFLUENCE ON OVERPRODUCTION OF THE AMINO ACIDS IN CORYNEBACTERIUM GLUTAMICUM"
- J BACTERIOL, JUL 1995, 177 (14) P4021-7, UNITED STATES, XP000675335 VRLJIC M ET AL: "Unbalance of L-lysine flux in Corynebacterium glutamicum and its use for the isolation of excretion-defective mutants."
- EUR. J. BIOCHEM., Bd. 202, 1991, Seiten 131-135, XP002037200 BRÖER S ET AL : "Lysine excretion by Corynebacterium glutamicum"

### SEQUENCE LISTING

<110> Forschungszentrum Jülich GmbH
<120> Process for the microbial production of amino acids by boosted activity of export carriers
<130> 1
<140> PCT/DE96/02485
   <141> 1996-12-18
<160> 5
<170> Patent In Ver. 2.0
<210> 1
   <211> 2374
   <212> DNA
   <213> Corynebacterium glutamicum
   <220> (LysE)
   <221> gene
   <222> CDS (1016)..(1726)
<400> 1
   ccatttgctg aaggtgttac tctgcctggc ccaattcctg cgggcgaaga agtgaaaaac 60
   cctgaacctt ttcagaagta actaaggccg caatccctcg attgctgcat caacgacggc 120
   gtctgtgagt ctagctagag atctagattc caggcgccat cgttgccaat acatcggtgt 180
   gtcaatgggt atctcatcga ggaggatcac ttctcctgct tttagcatgg gagcagcttg 240
   ggtttcggga agaagtcccc aaccaaggcc tcggcgaatt gcctcaccaa aaccttccgc 300
   cgacgggaca atggatacgc gcctgcgccc cacaggacca tcgacgcgcc cgtccaggtc 360
   acggtcttga agcacatctt tgggaccgaa gcgtaagacg ggcatcgcag cccaatctag 420
   tttcccatca accatgtagg catcccgcaa tgagggggtt gcaatggcca agtggcgcat 480
   ggttccaagt tctactactt cacatcccgc cacgggatta gcttcacggg ttaccgctcc 540
   taaaacatct ccacgccgca gcaaggataa tgtgtgcgct tcatcttcca agcgcagcgt 600
   gagcgttgct ccaccccaag aagctacctc gttgaacacg ggaggaaacc atgtggatag 660
   cgaatctgcg ttgatggcga tggttaacgg gatttcagca aggcgtccag atagttgcgc 720
   tttagtttct gcttgcagca acaccatttt ccgcgctgct tgcacaagga cttcacccgc 780
   ttcggttgct ttggccggtt gggtgcgcga taccaacact cgacccacgt gatgctcgag 840
   agctttaacg cgctgactca ccgccgaggg ggaaatggaa agggctaagg aggcgccttc 900
   gaagctgcct tcatcaatga ttgagagcaa agtgtccagt tgaatggggt tcatgaagct 960
   atattaaacc atgttaagaa ccaatcattt tacttaagta cttccatagg tcacg atg  1018
                                                                Met
                                                                 1
   gtg atc atg gaa atc ttc att aca ggt ctg ctt ttg ggg gcc agt ctt   1066
   Val Ile Met Glu Ile Phe Ile Thr Gly Leu Leu Leu Gly Ala Ser Leu
                   5                   10                  15
   tta ctg tcc atc gga ccg cag aat gta ctg gtg att aaa caa gga att    1114
   Leu Leu Ser Ile Gly Pro Gln Asn Val Leu Val Ile Lys Gln Gly Ile
               20                  25                  30
   aag cgc gaa gga ctc att gcg gtt ctt ctc gtg tgt tta att tct gac    1162
   Lys Arg Glu Gly Leu Ile Ala Val Leu Leu Val Cys Leu Ile Ser Asp
           35                  40                  45
   gtc ttt ttg ttc atc gcc ggc acc ttg ggc gtt gat ctt ttg tcc aat    1210
   Val Phe Leu Phe Ile Ala Gly Thr Leu Gly Val Asp Leu Leu Ser Asn
       50                  55                  60                  65
   gcc gcg ccg atc gtg ctc gat att atg cgc tgg ggt ggc atc gct tac    1258
   Ala Ala Pro Ile Val Leu Asp Ile Met Arg Trp Gly Gly Ile Ala Tyr
                       70                  75                  80
   ctg tta tgg ttt gcc gtc atg gca gcg aaa gac gcc atg aca aac aag    1306
   Leu Leu Trp Phe Ala Val Met Ala Ala Lys Asp Ala Met Thr Asn Lys
                   85                  90                  95
   gtg gaa gcg cca cag atc att gaa gaa aca gaa cca acc gtg ccc gat    1354
   Val Glu Ala Pro Gln Ile Ile Glu Glu Thr Glu Pro Thr Val Pro Asp
               100                 105                 110
   gac acg cct ttg ggc ggt tcg gcg gtg gcc act gac acg cgc aac cgg    1402
   Asp Thr Pro Leu Gly Gly Ser Ala Val Ala Thr Asp Thr Arg Asn Arg
           115                 120                 125
   gtg cgg gtg gag gtg agc gtc gat aag cag cgg gtt tgg gta aag ccc    1450
   Val Arg Val Glu Val Ser Val Asp Lys Gln Arg Val Trp Val Lys Pro
       130                 135                 140                 145
   atg ttg atg gca atc gtg ctg acc tgg ttg aac ccg aat gcg tat ttg    1498
   Met Leu Met Ala Ile Val Leu Thr Trp Leu Asn Pro Asn Ala Tyr Leu
                       150                 155                 160
   gac gcg ttt gtg ttt atc ggc ggc gtc ggc gcg caa tac ggc gac acc    1546
   Asp Ala Phe Val Phe Ile Gly Gly Val Gly Ala Gln Tyr Gly Asp Thr
                   165                 170                 175
   gga cgg tgg att ttc gcc gct ggc gcg ttc gcg gca agc ctg atc tgg    1594
   Gly Arg Trp Ile Phe Ala Ala Gly Ala Phe Ala Ala Ser Leu Ile Trp
               180                 185                 190
   ttc ccg ctg gtg ggt ttc ggc gca gca gca ttg tca cgc ccg ctg tcc    1642
   Phe Pro Leu Val Gly Phe Gly Ala Ala Ala Leu Ser Arg Pro Leu Ser
           195                 200                 205
   agc ccc aag gtg tgg cgc tgg atc aac gtc gtc gtg gca gtt gtg atg    1690
   Ser Pro Lys Val Trp Arg Trp Ile Asn Val Val Val Ala Val Val Met
       210                 215                 220                 225
   acc gca ttg gcc atc aaa ctg atg ttg atg ggt tag ttttcgcggg         1736
   Thr Ala Leu Ala Ile Lys Leu Met Leu Met Gly
                       230                 235
   ttttggaatc ggtggccttc gcccaaatgt tgatgccggc gtcgtgggaa atctcatcga 1796
   tcgcctccaa ctcggcgtca gaaaactcca agttgttgag tgaatcaagg ctgttgtcca 1856
   gctgctcaac tgacgaagca ccaatcaatg cactggtcac ggtatccgcg ccgtactctc 1916
   cttgctcgcg cagcacccat gcaagcgcca tctgcgcaag tgactgcccg cgttcctggg 1976
   cgatgtcatt gagcttgcgg accatatcaa tattgttcac gttcaacatg ccctcagaca 2036
   gggacttacc ctggctggcg cgggaaccct ctggaattcc atcgagatat ttgtccgtga 2096
   gcaggccctg cgcaagtggt gagaaagcaa tgacgccaag accattgttg gcagctgact 2156
   gcaacaagtt ctcaccgtca tcgcccggtt cctccaccca acgattaatg atggaatagc 2216
   ttggctgatg aatcagaagc gggcagccct cctccgccat gaactcagcc gcctccgctg 2276
   tgagctctgg accgtaggaa gaaataccca cgtaaagagc ctttccagac gcaacaatgt 2336
   cacgcaatgc gtacatggtt tcttccaaag gagtatct                         2374
<210> 2
   <211> 236
   <212> PRT
   <213> Corynebacterium glutamicum
   <220> (LysE)
<400> 2
   Met Val Ile Met Glu Ile Phe Ile Thr Gly Leu Leu Leu Gly Ala Ser
   1 5 10 15
   Leu Leu Leu Ser Ile Gly Pro Gln Asn Val Leu Val Ile Lys Gln Gly
   20 25 30
   Ile Lys Arg Glu Gly Leu Ile Ala Val Leu Leu Val Cys Leu Ile Ser
   35 40 45
   Asp Val Phe Leu Phe Ile Ala Gly Thr Leu Gly Val Asp Leu Leu Ser
   50 55 60
   Asn Ala Ala Pro Ile Val Leu Asp Ile Met Arg Trp Gly Gly Ile Ala
   65 70 75 80
   Tyr Leu Leu Trp Phe Ala Val Met Ala Ala Lys Asp Ala Met Thr Asn
   85 90 95
   Lys Val Glu Ala Pro Gln Ile Ile Glu Glu Thr Glu Pro Thr Val Pro
   100 105 110
   Asp Asp Thr Pro Leu Gly Gly Ser Ala Val Ala Thr Asp Thr Arg Asn
   115 120 125
   Arg Val Arg Val Glu Val Ser Val Asp Lys Gln Arg Val Trp Val Lys
   130 135 140
   Pro Met Leu Met Ala Ile Val Leu Thr Trp Leu Asn Pro Asn Ala Tyr
   145 150 155 160
   Leu Asp Ala Phe Val Phe Ile Gly Gly Val Gly Ala Gln Tyr Gly Asp
   165 170 175
   Thr Gly Arg Trp Ile Phe Ala Ala Gly Ala Phe Ala Ala Ser Leu Ile
   180 185 190
   Trp Phe Pro Leu Val Gly Phe Gly Ala Ala Ala Leu Ser Arg Pro Leu
   195 200 205
   Ser Ser Pro Lys Val Trp Arg Trp Ile Asn Val Val Val Ala Val Val
   210 215 220
   Met Thr Ala Leu Ala Ile Lys Leu Met Leu Met Gly
   225 230 235
<210> 3
   <211> 2374
   <212> DNA
   <213> Corynebacterium glutamicum
   <220> (complement to <210> 1)
   <221> unsure
   <222>CDS (2)..(652)
   <223>orf3
   <220>
   <221>gene
   <222>CDS (1421)..(2293)
   <223>LysG
<400> 3
   a gat act cct ttg gaa gaa acc atg tac gca ttg cgt gac att gtt gcg 49
     Asp Thr Pro Leu Glu Glu Thr Met Tyr Ala Leu Arg Asp Ile Val Ala
      1               5                  10                  15
   tct gga aag gct ctt tac gtg ggt att tct tcc tac ggt cca gag ctc   97
   Ser Gly Lys Ala Leu Tyr Val Gly Ile Ser Ser Tyr Gly Pro Glu Leu
                20                  25                  30
   aca gcg gag gcg gct gag ttc atg gcg gag gag ggc tgc ccg ctt ctg   145
   Thr Ala Glu Ala Ala Glu Phe Met Ala Glu Glu Gly Cys Pro Leu Leu
            35                  40                  45
   att cat cag cca agc tat tcc atc att aat cgt tgg gtg gag gaa ccg   193
   Ile His Gln Pro Ser Tyr Ser Ile Ile Asn Arg Trp Val Glu Glu Pro
        50                  55                  60
   ggc gat gac ggt gag aac ttg ttg cag tca gct gcc aac aat ggt ctt   241
   Gly Asp Asp Gly Glu Asn Leu Leu Gln Ser Ala Ala Asn Asn Gly Leu
    65                  70                  75                  80
   ggc gtc att gct ttc tca cca ctt gcg cag ggc ctg ctc acg gac aaa   289
   Gly Val Ile Ala Phe Ser Pro Leu Ala Gln Gly Leu Leu Thr Asp Lys
                    85                  90                  95
   tat ctc gat gga att cca gag ggt tcc cgc gcc agc cag ggt aag tcc   337
   Tyr Leu Asp Gly Ile Pro Glu Gly Ser Arg Ala Ser Gln Gly Lys Ser
               100                 105                 110
   ctg tct gag ggc atg ttg aac gtg aac aat att gat atg gtc cgc aag   385
   Leu Ser Glu Gly Met Leu Asn Val Asn Asn Ile Asp Met Val Arg Lys
           115                 120                 125
   ctc aat gac atc gcc cag gaa cgc ggg cag tca ctt gcg cag atg gcg   433
   Leu Asn Asp Ile Ala Gln Glu Arg Gly Gln Ser Leu Ala Gln Met Ala
       130                 135                 140
   ctt gca tgg gtg ctg cgc gag caa gga gag tac ggc gcg gat acc gtg    481
   Leu Ala Trp Val Leu Arg Glu Gln Gly Glu Tyr Gly Ala Asp Thr Val
   145                 150                 155                 160
   acc agt gca ttg att ggt gct tcg tca gtt gag cag ctg gac aac agc    529
   Thr Ser Ala Leu Ile Gly Ala Ser Ser Val Glu Gln Leu Asp Asn Ser
                   165                 170                 175
   ctt gat tca ctc aac aac ttg gag ttt tct gac gcc gag ttg gag gcg    577
   Leu Asp Ser Leu Asn Asn Leu Glu Phe Ser Asp Ala Glu Leu Glu Ala
               180                 185                 190
   atc gat gag att tcc cac gac gcc ggc atc aac att tgg gcg aag gcc    625
   Ile Asp Glu Ile Ser His Asp Ala Gly Ile Asn Ile Trp Ala Lys Ala
           195                 200                 205
   acc gat tcc aaa acc cgc gaa aac taa cccatcaaca tcagtttgat          672
   Thr Asp Ser Lys Thr Arg Glu Asn
       210                 215
   ggccaatgcg gtcatcacaa ctgccacgac gacgttgatc cagcgccaca ccttggggct 732
   ggacagcggg cgtgacaatg ctgctgcgcc gaaacccacc agcgggaacc agatcaggct 792
   tgccgcgaac gcgccagcgg cgaaaatcca ccgtccggtg tcgccgtatt gcgcgccgac 852
   gccgccgata aacacaaacg cgtccaaata cgcattcggg ttcaaccagg tcagcacgat 912
   tgccatcaac atgggcttta cccaaacccg ctgcttatcg acgctcacct ccacccgcac 972
   ccggttgcgc gtgtcagtgg ccaccgccga accgcccaaa ggcgtgtcat cgggcacggt 1032
   tggttctgtt tcttcaatga tctgtggcgc ttccaccttg tttgtcatgg cgtctttcgc 1092
   tgccatgacg gcaaaccata acaggtaagc gatgccaccc cagcgcataa tatcgagcac 1152
   gatcggcgcg gcattggaca aaagatcaac gcccaaggtg ccggcgatga acaaaaagac 1212
   gtcagaaatt aaacacacga gaagaaccgc aatgagtcct tcgcgcttaa ttccttgttt 1272
   aatcaccagt acattctgcg gtccgatgga cagtaaaaga ctggccccca aaagcagacc 1332
   tgtaatgaag atttccatga tcaccatcgt gacctatgga agtacttaag taaaatgatt 1392
   ggttcttaac atggtttaat atagcttc atg aac ccc att caa ctg gac act      1444
                                  Met Asn Pro Ile Gln Leu Asp Thr
                                              220                 225
   ttg ctc tca atc att gat gaa ggc agc ttc gaa ggc gcc tcc tta gcc    1492
   Leu Leu Ser Ile Ile Asp Glu Gly Ser Phe Glu Gly Ala Ser Leu Ala
                   230                 235                 240
   ctt tcc att tcc ccc tcg gcg gtg agt cag cgc gtt aaa gct ctc gag    1540
   Leu Ser Ile Ser Pro Ser Ala Val Ser Gln Arg Val Lys Ala Leu Glu
               245                 250                 255
   cat cac gtg ggt cga gtg ttg gta tcg cgc acc caa ccg gcc aaa gca    1588
   His His Val Gly Arg Val Leu Val Ser Arg Thr Gln Pro Ala Lys Ala
           260                 265                 270
   acc gaa gcg ggt gaa gtc ctt gtg caa gca gcg cgg aaa atg gtg ttg    1636
   Thr Glu Ala Gly Glu Val Leu Val Gln Ala Ala Arg Lys Met Val Leu
       275                 280                 285
   ctg caa gca gaa act aaa gcg caa cta tct gga cgc ctt gct gaa atc    1684
   Leu Gln Ala Glu Thr Lys Ala Gln Leu Ser Gly Arg Leu Ala Glu Ile
   290                 295                 300                 305
   ccg tta acc atc gcc atc aac gca gat tcg cta tcc aca tgg ttt cct    1732
   Pro Leu Thr Ile Ala Ile Asn Ala Asp Ser Leu Ser Thr Trp Phe Pro
                   310                 315                 320
   ccc gtg ttc aac gag gta gct tct tgg ggt gga gca acg ctc acg ctg    1780
   Pro Val Phe Asn Glu Val Ala Ser Trp Gly Gly Ala Thr Leu Thr Leu
               325                 330                 335
   cgc ttg gaa gat gaa gcg cac aca tta tcc ttg ctg cgg cgt gga gat    1828
   Arg Leu Glu Asp Glu Ala His Thr Leu Ser Leu Leu Arg Arg Gly Asp
           340                 345                 350
   gtt tta gga gcg gta acc cgt gaa gct aat ccc gtg gcg gga tgt gaa    1876
   Val Leu Gly Ala Val Thr Arg Glu Ala Asn Pro Val Ala Gly Cys Glu
       355                 360                 365
   gta gta gaa ctt gga acc atg cgc cac ttg gcc att gca acc ccc tca    1924
   Val Val Glu Leu Gly Thr Met Arg His Leu Ala Ile Ala Thr Pro Ser
   370                 375                 380                 385
   ttg cgg gat gcc tac atg gtt gat ggg aaa cta gat tgg gct gcg atg    1972
   Leu Arg Asp Ala Tyr Met Val Asp Gly Lys Leu Asp Trp Ala Ala Met
                   390                 395                 400
   ccc gtc tta cgc ttc ggt ccc aaa gat gtg ctt caa gac cgt gac ctg    2020
   Pro Val Leu Arg Phe Gly Pro Lys Asp Val Leu Gln Asp Arg Asp Leu
               405                 410                 415
   gac ggg cgc gtc gat ggt cct gtg ggg cgc agg cgc gta tcc att gtc    2068
   Asp Gly Arg Val Asp Gly Pro Val Gly Arg Arg Arg Val Ser Ile Val
           420                 425                 430
   ccg tcg gcg gaa ggt ttt ggt gag gca att cgc cga ggc ctt ggt tgg    2116
   Pro Ser Ala Glu Gly Phe Gly Glu Ala Ile Arg Arg Gly Leu Gly Trp
       435                 440                 445
   gga ctt ctt ccc gaa acc caa gct gct ccc atg cta aaa gca gga gaa    2164
   Gly Leu Leu Pro Glu Thr Gln Ala Ala Pro Met Leu Lys Ala Gly Glu
   450                 455                 460                 465
   gtg atc ctc ctc gat gag ata ccc att gac aca ccg atg tat tgg caa    2212
   Val Ile Leu Leu Asp Glu Ile Pro Ile Asp Thr Pro Met Tyr Trp Gln
                   470                 475                 480
   cga tgg cgc ctg gaa tct aga tct cta gct aga ctc aca gac gcc gtc    2260
   Arg Trp Arg Leu Glu Ser Arg Ser Leu Ala Arg Leu Thr Asp Ala Val
               485                 490                 495
   gtt gat gca gca atc gag gga ttg cgg cct tag ttacttctga aaaggttcag 2313
   Val Asp Ala Ala Ile Glu Gly Leu Arg Pro
           500                 505
   ggtttttcac ttcttcgccc gcaggaattg ggccaggcag agtaacacct tcagcaaatg 2373
   g                                                                 2374
<210> 4
   <211> 216
   <212> PRT
   <213> Corynebacterium glutamicum
   <220> (orf3)
<400> 4
   Asp Thr Pro Leu Glu Glu Thr Met Tyr Ala Leu Arg Asp Ile Val Ala
    1               5                  10                  15
   Ser Gly Lys Ala Leu Tyr Val Gly Ile Ser Ser Tyr Gly Pro Glu Leu
               20                  25                  30
   Thr Ala Glu Ala Ala Glu Phe Met Ala Glu Glu Gly Cys Pro Leu Leu
           35                  40                  45
   Ile His Gln Pro Ser Tyr Ser Ile Ile Asn Arg Trp Val Glu Glu Pro
       50                  55                  60
   Gly Asp Asp Gly Glu Asn Leu Leu Gln Ser Ala Ala Asn Asn Gly Leu
   65                  70                  75                  80
   Gly Val Ile Ala Phe Ser Pro Leu Ala Gln Gly Leu Leu Thr Asp Lys
                   85                  90                  95
   Tyr Leu Asp Gly Ile Pro Glu Gly Ser Arg Ala Ser Gln Gly Lys Ser
               100                 105                 110
   Leu Ser Glu Gly Met Leu Asn Val Asn Asn Ile Asp Met Val Arg Lys
           115                 120                 125
   Leu Asn Asp Ile Ala Gln Glu Arg Gly Gln Ser Leu Ala Gln Met Ala
       130                 135                 140
   Leu Ala Trp Val Leu Arg Glu Gln Gly Glu Tyr Gly Ala Asp Thr Val
   145                 150                 155                 160
   Thr Ser Ala Leu Ile Gly Ala Ser Ser Val Glu Gln Leu Asp Asn Ser
                   165                 170                 175
   Leu Asp Ser Leu Asn Asn Leu Glu Phe Ser Asp Ala Glu Leu Glu Ala
               180                 185                 190
   Ile Asp Glu Ile Ser His Asp Ala Gly Ile Asn Ile Trp Ala Lys Ala
           195                 200                 205
   Thr Asp Ser Lys Thr Arg Glu Asn
       210                 215
<210> 5
   <211> 290
   <212> PRT
   <213> Corynebacterium glutamicum
   <220> (LysG)
<400> 5
   Met Asn Pro Ile Gln Leu Asp Thr Leu Leu Ser Ile Ile Asp Glu Gly
    1               5                  10                  15
   Ser Phe Glu Gly Ala Ser Leu Ala Leu Ser Ile Ser Pro Ser Ala Val
               20                  25                  30
   Ser Gln Arg Val Lys Ala Leu Glu His His Val Gly Arg Val Leu Val
           35                  40                  45
   Ser Arg Thr Gln Pro Ala Lys Ala Thr Glu Ala Gly Glu Val Leu Val
       50                  55                  60
   Gln Ala Ala Arg Lys Met Val Leu Leu Gln Ala Glu Thr Lys Ala Gln
   65                  70                  75                  80
   Leu Ser Gly Arg Leu Ala Glu Ile Pro Leu Thr Ile Ala Ile Asn Ala
                   85                  90                  95
   Asp Ser Leu Ser Thr Trp Phe Pro Pro Val Phe Asn Glu Val Ala Ser
               100                 105                 110
   Trp Gly Gly Ala Thr Leu Thr Leu Arg Leu Glu Asp Glu Ala His Thr
           115                 120                 125
   Leu Ser Leu Leu Arg Arg Gly Asp Val Leu Gly Ala Val Thr Arg Glu
       130                 135                 140
   Ala Asn Pro Val Ala Gly Cys Glu Val Val Glu Leu Gly Thr Met Arg
   145                 150                 155                 160
   His Leu Ala Ile Ala Thr Pro Ser Leu Arg Asp Ala Tyr Met Val Asp
                   165                 170                 175
   Gly Lys Leu Asp Trp Ala Ala Met Pro Val Leu Arg Phe Gly Pro Lys
               180                 185                 190
   Asp Val Leu Gln Asp Arg Asp Leu Asp Gly Arg Val Asp Gly Pro Val
           195                 200                 205
   Gly Arg Arg Arg Val Ser Ile Val Pro Ser Ala Glu Gly Phe Gly Glu
       210                 215                 220
   Ala Ile Arg Arg Gly Leu Gly Trp Gly Leu Leu Pro Glu Thr Gln Ala
   225                 230                 235                 240
   Ala Pro Met Leu Lys Ala Gly Glu Val Ile Leu Leu Asp Glu Ile Pro
                   245                 250                 255
   Ile Asp Thr Pro Met Tyr Trp Gln Arg Trp Arg Leu Glu Ser Arg Ser
               260                 265                 270
   Leu Ala Arg Leu Thr Asp Ala Val Val Asp Ala Ala Ile Glu Gly Leu
           275                 280                 285
   Arg Pro
       290

## Beschreibung

Die Erfindung betrifft ein Verfahren zur mikrobiellen Her-stellung von Aminosäuren gemäß den Ansprüchen 1 bis 20, Ex-portgene nach Anspruch 21 bis 26, Regulatorgene nach Anspruch 27 und 28, Genstrukturen gemäß den Ansprüchen 29 und 30, Vektoren nach Anspruch 31 bis 33, transformierte Zellen nach Anspruch 34 bis 40, Membran-proteine gemäß Anspruch 41 und 42 sowie Verwendungen nach Anspruch 43 bis 48.

Aminosäuren sind von großem wirtschaftlichen Interesse, wobei die Verwendung von Aminosäuren vielfältig ist: So wird z.B. L-Lysin, wie auch L-Threonin, und L-Tryptophan als Futtermittelzusatz benötigt, L-Glutamat als Gewürzzusatz, L-Isoleucin und L-Tyrosin in der pharmazeutischen Industrie, L-Arginin und L-Isoleucin als Medikament, oder L-Glutamat und L-Phenylalanin als Ausgangssubstanz zur Synthese von Feinchemikalien.

Eine bevorzugte Methode zur Herstellung dieser verschiedensten Aminosäuren ist die biotechnologische Herstellung mittels Mikroorganismen; denn auf diese Weise wird direkt die biologisch wirksame und optisch aktive Form der jeweiligen Aminosäure erhalten, und es können einfache und preisgünstige Rohstoffe eingesetzt werden. Als Mikroorganismen werden z.B. Corynebacterium glutamicum und seine Verwandten ssp. flavum und ssp. lactofermentum (Liebl et al.,Int J System Bacteriol (1991) 41:255-260) wie auch Escherichia coli und verwandte Bakterien eingesetzt.

Diese Bakterien produzieren die Aminosäuren normalerweise aber nur in der zum Wachstum benötigten Menge, so daß also keine überschüßigen Aminosäuren gebildet und ausgeschieden werden. Dies ist darin begründet, daß in der Zelle die Biosynthese der Aminosäuren in vielfacher Weise kontrolliert wird. Folglich sind bereits verschiedenste Verfahren bekannt, um die Produktbildung durch Ausschaltung der Kontrollmechanismen zu steigern. Bei diesen Prozessen werden z.B. Aminosäureanaloga eingesetzt, um die effektive Regulation der Biosynthese auszuschalten. So ist ein Verfahren beschrieben, bei dem Corynebacterium-Stämme benutzt werden, die gegen L-Tyrosin- und L-Phenylalaninanaloga resistent sind (JP 19037/1976 und 39517/1978). Ebenso sind Verfahren beschrieben, bei denen gegenüber L-Lysin- oder auch L-Threoninanaloga resistente Bakterien eingesetzt werden, um die Kontrollmechanismen zu überwinden (EP 0 205 849 B1, UK Patent Application GB 2 152 509 A).

Weiterhin sind auch durch rekombinante DNA-Techniken konstruierte Mikroorganismen bekannt, bei denen ebenfalls die Regulation der Biosynthese aufgehoben ist, indem die Gene, die für die nicht mehr feedbackinhibierbaren Schlüsselenzyme kodieren, kloniert und exprimiert werden. So ist z.B. ein rekombinantes, L-Lysin produzierendes Bakterium mit plasmid-kodierter, feedback-resistenter Aspartatkinase bekannt (EP 0 381 527). Ebenso ist ein rekombinantes, L-Phenylalanin produzierendes Bakterium mit feedback-resistenter Prephenatdehydrogenase beschrieben (JP 124375/1986, EP 0 488 424). Darüber hinaus wurden auch durch Überexpression von Genen, die nicht für feedback-sensitive Enzyme der Aminosäuresynthese codieren, erhöhte Aminosäureausbeuten erreicht. So wird z.B. die Lysinbildung durch erhöhte Synthese der Dihydrodipicolinatsynthase verbessert (EP 0 197 335). Ebenso wird durch erhöhte Synthese der Threonindehydratase eine verbesserte Threoninbildung erreicht (EP 0 436 886 A1).

Weitere Versuche zur Erhöhung der Aminosäureproduktion zielen auf eine verbesserte Bereitstellung der zellulären Primär-metabolite des Zentralstoffwechsels. So ist bekannt, daß die durch rekombinante Techniken erreichte Überexpression der Transketolase eine verbesserte Produktbildung von L-Tryptophan, L-Tyrosin, oder L-Phenylalanin ermöglicht (EP 0 600 463 A2). Weiterhin führt die Reduktion der Phosphoenolpyruvatcarboxylase-Aktivität in Corynebacterium zu verbesserter Bildung aromatischer Aminosäuren (EP 0 3331 145).

Diese vielfältigen Versuche zur Produktivitätssteigerung sind insgesamt darauf gerichtet, die Limitation der cytosolischen Synthese der Aminosäuren zu überwinden. Als eine weitere Limitation kommt grundsätzlich aber auch der Export der im Zellinneren gebildeten Aminosäuren ins Kulturmedium in Be-tracht. Daher gibt es vereinzelte Ansätze, diesen Export und damit die Wirtschaftlichkeit der Aminosäureproduktion zu verbessern. So hat man die Zellpermeabilität bei Coryne-bacterium durch Biotinmangel, Detergenz- oder Penicillinbehandlung erhöht. Diese Ausschleusehilfen waren jedoch ausschließlich bei der Glutamatproduktion erfolgreich, während die Synthese anderer Aminosäuren auf diese Weise nicht verbessert werden konnte. Auch sind Bakterienstämme entwickelt worden, bei denen die Aktivität des Sekretionssystems aufgrund chemischer oder physikalischer Mutation erhöht ist. Es wurde dadurch beispielsweise ein Corynebacterium glutamicum-Stamm erhalten, der sich durch eine verbesserte Sekretionsaktivität insbesondere für die L-Lysinproduktion eignet (DE 42 03 320).

Insgesamt zeichnen sich alle bisher durchgeführten Versuche zur Erhöhung der Sekretion zellintern gebildeter Aminosäuren dadurch aus, daß ein erhöhter Efflux von Aminosäuren aufgrund der gewählten ungerichteten bzw. unspezifischen Methoden nur durch Zufall erreicht werden konnte. Einzig in der Deutschen Patentanmeldung No. 195 23 279.8-41 ist ein Verfahren beschrieben, das es erlaubt, die Sekretion zellintern gebildeter Aminosäuren ganz gezielt zu erhöhen, indem die Expression von für den Import von Aminosäuren kodierenden Genen erhöht wurde. Die dieser Vorgehensweise zugrundeliegende Erkenntnis, daß die Zelle Importproteine für den Export von Aminosäuren verwendet wie auch die Tatsache, daß Mikroorganismen von Natur aus keine überschüssigen Aminosäuren bilden und ausscheiden, legt die Vermutung nahe, daß für den Aminosäuretransport spezifische Exportgene bzw. -proteine gar nicht existieren, sondern daß aus der Zelle die Aminosäuren über andere Exportsysteme exkretiert werden.

Die bisher bekannten Exportsysteme exportieren giftige Metallionen, toxische Antibiotika und höhermolekulare Toxine. Diese Exportsysteme sind relativ komplex aufgebaut: In der Regel sind Membranproteine der Cytoplasmamembran beteiligt, die jedoch nur eine Teilreaktion des Exports bewirken, so daß vermutlich für den Transport zusätzliche, extracytoplasmatische Hilfsproteine erforderlich sind (Dinh, T. et al., A family of extracytoplasmic proteins that allow transport of large molecules across the outer membranes of gram-negative bacteria. J. Bacteriol. 1994, 176: 3825-3831). Des weiteren ist bekannt, daß bei dem sec-abhängigen Exportsystem für extrazelluläre Proteine mindestens 6 verschiedene Proteinkomponenten für den Export essentiell sind. Dieser Stand der Technik legt die Vermutung nahe, daß ebenso die für den Export von Aminosäuren zuständigen, aber bislang unbekannten Systeme aus mehreren Proteinkomponenten bestehen bzw. mehrere Gene für den Export von Aminosäuren zuständig sind. Hinweis dafür könnten die von Vrljic et al. beschriebenen (J Bacteriol (1995) 177: 4021-4027) verschiedenen, im Lysinexport defekten Mutanten sein.

Es wurde nunmehr überraschenderweise gefunden, daß für den Export von Aminosäuren jeweils nur ein einziges, spezifisches Gen verantwortlich ist, so daß erfindungsgemäß erstmals ein Verfahren zur mikrobiellen Herstellung von Aminosäuren zur Verfügung gestellt wird, bei dem gezielt die Exportgen-Expression und/oder die Exportcarrier-Aktivität eines die entsprechende Aminosäure produzierenden Mikroorganismus erhöht wird. Die aus dieser Verfahrensweise resultierende, gesteigerte Expression bzw. Aktivität des Exportcarriers führt zu einer erhöhten Sekretionsrate, so daß der Export der entsprechenden Aminosäure erhöht ist. Auch akkumulieren derart veränderte Mikroorganismen einen erhöhten Anteil der entsprechenden Aminosäure im Kulturmedium.

Zur Erhöhung der Exportcarrier-Aktivität wird insbesondere die endogene Aktivität eines Aminosäureproduzierenden Mikroorganismus erhöht. Eine Erhöhung der Enzymaktivität kann beispielsweise erreicht werden, indem durch Veränderung des katalytischen Zentrums ein erhöhter Substratumsatz erfolgt oder indem die Wirkung von Enzyminhibitoren aufgehoben wird. Auch kann eine erhöhte Enzymaktivität durch Erhöhung der Enzymsynthese, beispielsweise durch Genamplifikationen oder durch Ausschaltung von Faktoren, die die Enzym-Biosynthese reprimieren, hervorgerufen werden. Die endogene Exportcarrier-Aktivität wird vorzugsweise durch Mutation des endogenen Exportgens erhöht. Derartige Mutationen können entweder nach klassischen Methoden ungerichtet erzeugt werden, wie beispielsweise durch UV-Bestrahlung oder mutationsauslösenden Chemikalien, oder gezielt mittels gentechnologischer Methoden wie Deletion(en), Insertion(en) und/oder Nukleotidaustausch(e).

Die Exportgen-Expression wird durch Erhöhen der Genkopienzahl und/oder durch Verstärkung regulatorischer Faktoren, die die Exportgen-Expression positiv beeinflussen, erhöht. So kann eine Verstärkung regulatorischer Elemente vorzugsweise auf der Transkriptionsebene erfolgen, indem insbesondere die Transkriptionssignale erhöht werden. Dies kann beispielsweise dadurch erfolgen, daß durch Veränderung der dem Strukturgen vorgeschalteten Promotorsequenz der Promotor in seiner Wirksamkeit erhöht wird oder indem der Promotor komplett durch wirksamere Promotoren ausgetauscht wird. Auch kann eine Verstärkung der Transkription durch entsprechende Beeinflußung eines dem Exportgen zugeordneten Regulatorgens erfolgen, wie weiter unten ausgeführt wird. Daneben ist aber auch eine Verstärkung der Translation möglich, indem beispielsweise die Stabilität der m-RNA verbessert wird.

Zur Erhöhung der Genkopienzahl wird das Exportgen in ein Genkonstrukt bzw. in einen Vektor eingebaut, vorzugsweise in einen Vektor mit niedriger Kopienzahl. Das Genkonstrukt enthält insbesondere dem Exportgen zugeordnete regulatorische Gensequenzen, vorzugsweise solche, die die Genexpression verstärken. Die regulatorischen Gensequenzen weisen insbesondere eine für die in Tabelle 1 angegebene Aminosäuresequenz oder deren Allelvariationen kodierende Nukleotidsequenz bzw. eine Nukleotidsequenz von Nukleotid 954 bis 82 gemäß Tabelle 2 oder eine im wesentlichen gleichwirkende DNA-Sequenz auf. Allelvariationen bzw. gleichwirkende DNA-Sequenzen umfassen insbesondere funktionelle Derivate, die durch Deletion(en), Insertion(en) und/oder Substitution(en) von Nukleotiden aus entsprechenden Sequenzen erhältlich sind, wobei aber die Regulatorprotein-Aktivität bzw. -Funktion erhalten bleibt oder sogar erhöht ist: So kann durch Mutation der regulatorischen Gensequenz die Effektivität der Bindung des Regulatorproteins an die DNA des zu regulierenden Exportgens so beeinflußt sein, daß dadurch die Transkription verstärkt und somit die Genexpression erhöht ist. Des weiteren können dem Exportgen als regulatorische Sequenzen aber auch sog. "enhancer" zugeordnet sein, die über eine verbesserte Wechselwirkung zwischen RNA-Polymerase und DNA ebenfalls eine erhöhte Exportgen-Expression bewirken.

Für den Einbau des Exportgens in ein Genkonstrukt wird das Exportgen vorzugsweise aus einem Mikroorganismen-Stamm der Gattung Corynebacterium isoliert, und mit dem das Exportgen enthaltende Genkonstrukt ein die entsprechende Aminosäure produzierender Mikroorganismen-Stamm, insbesondere Corynebacterium, transformiert. Die Isolierung und Transformation des entsprechenden Transportgens erfolgt nach gängigen Methoden: Im Falle der Isolierung und Klonierung eines Transportgens aus Corynebacterium eignet sich beispielsweise die Methode der homologen Komplementation einer exportdefekten Mutante (J Bacteriol (1995) 177: 4021-4027). Falls keine direkte Klonierung des Strukturgens möglich ist, kann zunächst auch die Insertion von Vektorsequenzen in das Transportgen erfolgen, um es dann über "plasmid-rescue"in Form inaktiver Fragmente zu isolieren. Für das erfindungsgemäße Verfahren eignen sich insbesondere Gene aus C. glutamicum ATCC 13032 oder C. glutamicum ssp. flavum ATCC 14067 oder auch C. glutamicum ssp. lactofermentum ATCC 13869. Nach Isolierung der Gene und deren in vitro-Rekombination mit bekannten Vektoren (Appl Env Microbiol (1989) 55: 684-688; Gene 102 (1991) 93-98), erfolgt die Transformation in die Aminosäureproduzierenden Stämme durch Elektroporation (Liebl et al. (1989) FEMS Microbiol Lett 65: 299-304) oder Konjugation (Schäfer et al. (1990) J Bacteriol 172: 1663-1666). Für die Übertragung werden vorzugsweise Vektoren mit niedriger Kopienzahl eingesetzt. Als Wirtszellen werden vorzugsweise solche Aminosäureproduzenten eingesetzt, die in der Synthese der entsprechenden Aminosäuren dereguliert sind und/oder die einen erhöhten Anteil an Zentralstoffwechselmetaboliten enthalten.

Nach Isolierung sind Exportgene mit Nukleotidsequenzen erhältlich, die für die in Tabelle 3 angegebene Aminosäuresequenz oder deren Allelvariationen kodieren bzw. die die Nukleotidsequenz von Nukleotid 1016 bis 1726 gemäß Tabelle 2 oder eine im wesentlichen gleichwirkende DNA-Sequenz aufweisen. Auch hier umfassen Allelvariationen bzw. gleichwirkende DNA-Sequenzen insbesondere funktionelle Derivate im oben für die regulatorischen Sequenzen angegebenen Sinne. Diese Exportgene werden vorzugsweise im erfindungsgemäßen Verfahren eingesetzt.

Dem Exportgen mit oder ohne vorgeschaltetem Promotor bzw. mit oder ohne zugeordnetem Regulatorgen können ein oder mehrere DNA-Sequenzen vor- und/oder nachgeschaltet sein, so daß das Gen in einer Genstruktur enthalten ist.

Durch Klonierung von Exportgenen sind Plasmide bzw. Vektoren erhältlich, die das Exportgen enthalten und - wie bereits oben erwähnt - zur Transformation eines Aminosäure-Produzenten geeignet sind. Die durch Transformation erhältlichen Zellen, bei denen es sich vorzugsweise um transformierte Zellen von Corynebacterium handelt, enthalten das Gen in replizierbarer Form, d.h. in zusätzlichen Kopien auf dem Chromosom, wobei die Genkopien durch homologe Rekombination an beliebigen Stellen des Genoms integriert werden, und/oder auf einem Plasmid bzw. Vektor.

Es sind eine Vielzahl von Sequenzen bekannt, die für Membran-proteine unbekannter Funktion kodieren. Durch die erfindungs-gemäße Bereitstellung von Exportgenen, wie beispielsweise des Exportgens mit der Nukleotidsequenz von Nukleotid 1016 bis 1726 gemäß Tabelle 2, bzw. der entsprechenden Exportproteine, wie z.B. das mit der Aminosäuresequenz gemäß Tabelle 1, können nunmehr Membranproteine, deren Funktion der Transport von Aminosäuren ist, durch Sequenzvergleich identifiziert werden. Das damit identifizierte Exportgen kann anschließend zur Verbesserung der Aminosäureproduktion nach dem erfin-dungsgemäßen Verfahren eingesetzt werden.

Die aus dem Stand der Technik bekannten Membranproteine be-sitzen in der Regel 12, zum Teil auch 4 transmembrane Helices. Es wurde nunmehr überraschenderweise gefunden, daß die für den Export von Aminosäuren zuständigen bzw. geeigneten Membranproteine 6 transmembrane Helices aufweisen (vgl. z.B. die in Tabelle 3 aufgeführte Aminosäuresequenz eines Exportproteins, bei der die 6 transmembranen Bereiche durch Unterstreichen kenntlich gemacht sind). Damit liegt hier eine bisher noch nicht beschriebene und somit neue Klasse von Membranproteinen vor.

### Ausführungsbeispiele

### a) Klonierung eines Exportgens und Klonierung eines Regulators aus Corynebacterium glutamicum

Chromosomale DNA aus C. glutamicum R127 (FEMS Microbiol Lett (1989) 65: 299-304) wurde, wie bei Scharzer et al. (Bio/Technology (1990) 9: 84-87) beschrieben, isoliert. Diese wurde mit dem Restriktionsenzym Sau3A gespalten und durch Saccharose-Gradienten-Zentrifugation, wie bei Sambrook et al. (Molecular Cloning, A laboratory manual (1989) Cold Spring Harbour Laboratory Press) beschrieben, aufgetrennt. Die einzelnen Fraktionen wurden gelelektrophoretisch auf ihre Größe hin analysiert und die Fraktion mit einer Fragmentgröße von etwa 6-10 kb zur Ligation mit dem Vektor pJC1 eingesetzt. Dazu wurde der Vektor pJC1 mit BamHI linearisiert und dephosphoryliert. Fünf ng davon wurde mit 20 ng der chromosomalen 6-10 kb Fragmente ligiert. Mit dem gesamten Ligationsansatz wurde die exportdefekte Mutante NA8 (J Bacteriol (1995) 177: 4021-4027) durch Elektroporation (FEMS Microbiol Lett (1989) 65: 299-304) transformiert. Die Transformanten wurden auf LBHIS (FEMS Microbiol Lett (1989) 65: 299-304) mit 15 *µ*g Kanamycin pro ml selektioniert. Diese Transformanten wurden umfangreichen Plasmidanalysen unterzogen, indem 200 der insgesamt 4500 erhaltenen Klone einzeln angezogen, und deren Plasmidanteil, und -größe bestimmt wurden. Im Durchschnitt trug etwa die Hälfte der untersuchten Kanamycin-resistenten Klone ein rekombinantes Plasmid mit einem Insert der durchschnittlichen Größe von 8 kb. Damit ergibt sich eine Wahrscheinlichkeit von 0,96 für die Anwesenheit jedes x-beliebigen Gens aus C. glutamicum in der errichteten Genbank. Die 4500 erhaltenen Transformanten wurden alle einzeln auf Wiedererhalt der Lysinsekretion geprüft. Dazu wurde das von Vrljic beschriebene System zur Induktion der L-Lysinausscheidung in Corynebacterium glutamicum eingesetzt (J Bacteriol (1995) 177: 4021-4027). Dazu wurden sogenannte Minimalmedium-Indikatorplatten hergestellt, die pro Liter 20 g (NH₄)₂SO₄, 5 g Harnstoff, 1 g KH₂PO₄, 1 g K₂HPO₄, 0,25 g MgSO₄ x 7 H₂O, 42 g Morpholinopropansulfonsäure, 1 ml CaCl₂ (1 g/100 ml), 750 ml dest., 1ml Cg Spuren-salze, 1 ml Biotin (20 mg/100 ml), pH7, 4 % Glukose 1,8 mg Protokatechusäure, 1 mg FeSO₄ x 7 H₂O, 1 mg MnSO₄ x H₂O, 0,1 mg ZnSO₄ x 7 H₂O, 0,02 mg CuSO₄, 0,002 mg NiCl₂ 6 H₂O, 20 g Agar-Agar, sowie 10⁷ Zellen/ml der Lysin-auxotrophen C. glutamicum Mutante 49/3 enthielten. Die ursprünglichen 4500 Transformanten wurden alle einzeln mittels Zahnstocher auf die Indikatorplatten gepickt, mit jeweils einer Kontrolle des ursprünglichen Nichtausscheiders NA8 (J Bacteriol (1995) 177: 4021-4027) und des Ausgangsstammes R127. Parallel wurden jeweils 2 Platten beimpft, von denen nur eine zusätzlich 5 mM L-Methionin enthielt, um so die Lysinausscheidung zu induzieren. Die Indikatorplatten wurden bei 30 °C inkubiert, und nach 15, 24 und 48 Stunden untersucht. Insgesamt wurden so 29 Klone erhalten, die auf der mit Methionin versetzten Indikator-platte einen Wachstumshof durch den Indikationsstamms 49/3 zeigten. Die Klone wurden vereinzelt, und dann erneut, wie oben beschrieben, auf Wiedererhalt des Wachstumshofs geprüft. Auf diese Weise wurden die zwei Klone NA8 pMV8-5-24 und NA8 pMV6-3 erhalten, die die Fähigkeit wiedererhalten hatten, Lysin auszuscheiden.

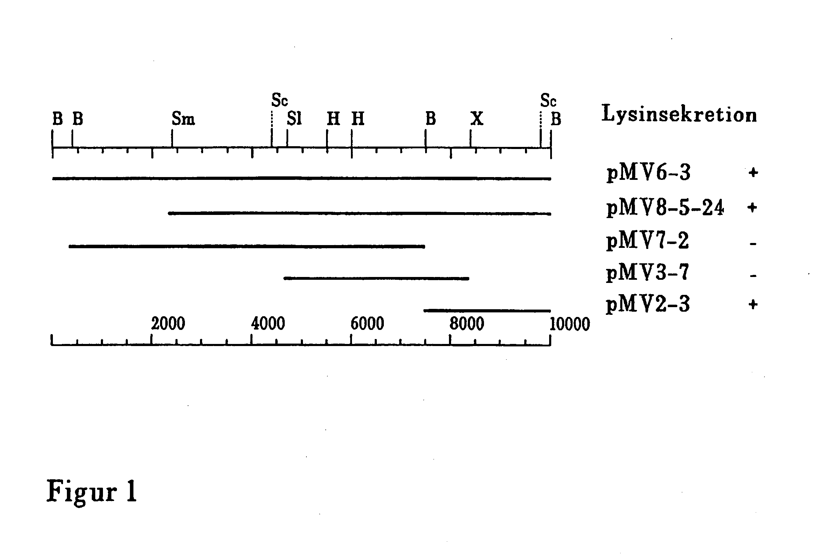

Von diesen Klonen wurden Plasmidpräparationen, wie bei Schwarzer et al. (Bio/Technology (1990) 9: 84-87) beschrieben, durchgeführt. Durch Retransformation in NA8 wurde der plasmidgebundene Effekt der Auscheidung von L-Lysin bestätigt. Beide Plasmide wurden einer Restriktionsanalyse unterzogen. Plasmid pMV8-5-24 trägt ein Insert von 8,3 kb, und pMV6-3 eines von 9,5 kb. Die physikalische Kartierung der Inserts zeigt Figur 1.

### b) Subklonierung eines DNA-Fragments, das den Lysinexport rekonstituiert

Vom Insert des Plasmids pMV6-3 wurden unter Nutzung der bestimmten Restriktionsschnittstellen einzelne Subklone hergestellt. So wurde das 3,7 kb XhoI-SalI-Fragment, das 2,3 kb BamHI-Fragment und das 7,2 kb BamHI-Fragment mit dem entsprechend geschnittenem und behandeltem Vektor pJC1 (Mol Gen Genet (1990) 220: 478-480) ligiert. Mit den Ligationsprodukten wurde direkt C. glutamicum NA8 transformiert, die Transformanten wie oben beschrieben auf Wiedererhalt der Lysinausscheidung geprüft und die Anwesenheit des Subklons durch Plasmidpräparation und Restriktionsanalyse bestätigt. Auf diese Weise wurde als kleinster Subklon der Stamm mit Plasmid pMV2-3 erhalten (Figur 1). Dieses, den Lysinexport vermittelnde Fragment enthält als Insert das 2,3 kb BamHI-Fragment aus pMV6-3.

### c) Sequenz des Lysinexportgens lysE und dessen Regulators lysG

Die Nukleotidsequenz des 2,3 kb BamHI-Fragments wurde nach der Dideoxy-Kettenabbruch-Methode von Sanger et al. durchgeführt (Proc Natl Acad Sci USA (1977) 74: 5463-5467), und die Sequenziereaktionen mit dem Auto-Read Sequencing kit von Pharmacia (Uppsala, Sweden). Die elektrophoretische Analyse erfolgte mit dem automatischen Laser-Flureszenz DNA Sequenziergerät (A.L.F.) von Pharmacia-LKB (Piscataway, NJ, USA). Die erhaltene Nukleotidsequenz wurde mit dem Programmpaket HUSAR (Release 3.0) des Deutschen Krebsforschungszentrums (Heidelberg) analysiert. Die Nukleotidsequenz und das Ergebnis der Analyse ist in Tabelle 2 wiedergegeben. Die Analyse ergibt zwei vollständige offene Leseraster (ORF) auf dem sequenzierten DNA-Stück. ORF1 kodiert für ein Protein mit einer Länge von 236 Aminosäuren, ORF2 für eins mit einer Länge von 290 Aminosäuren. Das von ORF1 abgeleitete Protein zeigt eine Häufung hydrophober Aminosäuren, wie sie für membranständige Proteine charakteristisch ist. Die detaillierte Analyse der Verteilung der hydrophoben und hydrophilen Aminosäuren mit dem Programm PHD.HTM (Protein Science (1995) 4: 521-533) ist in Tabelle 3 gezeigt. Daraus ergibt sich, daß das Protein sechs hydrophobe Helixbereiche enthält, die die Membran durchqueren. Damit handelt es sich bei diesem Protein um den gesuchten Exporter der Aminosäure L-Lysin. Das entsprechende Gen wird deswegen im Folgenden als lysE bezeichnet. Es ist entsprechend in Tabelle 2 markiert. ORF2 wird in Gegenrichtung zu ORF1 transkribiert. Die Sequenzanalyse zeigt, daß ORF2 hohe Identität mit Regulatorgenen hat, die als eine Familie zusammengefaßt werden (Ann Rev Microbiol (1993) 597-626). Gene dieser Familie regulieren die Expression der verschiedensten an katabolen oder anabolen Prozessen beteiligter Gene in positiver Weise. Im Folgenden wird ORF2 deswegen als lysG (Govern = Regulieren) bezeichnet. Wegen dieser Zuordnung, und weil lysE nur zusammen mit lysG kloniert (siehe a)) und subkloniert werden konnte (siehe b)), ist lysG Regulator von lysE und somit ebenfalls am Lysinexport beteiligt. Das Gen lysG und dessen abgeleitete Aminosäuresequenz sind ebenfalls in Tabelle 2 bzw. Tabelle 1 gezeigt.

### d) Identifizierung eines unbekannten Membranproteins aus Escherichia coli durch Sequenzvergleich

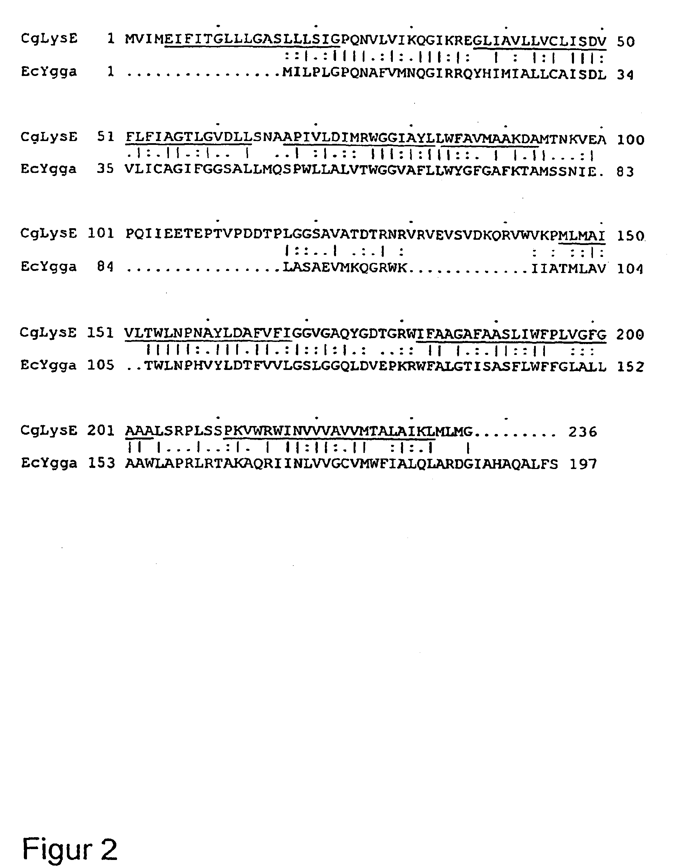

Mit den etablierten Sequenzen gemäß Tabelle 3 können bereits existierende Sequenzbanken durchsucht werden, um so den von sequenzierten Bereichen abgeleiteten Proteinen eine Funktion zuzuordnen. Entsprechend wurde die Aminosäuresequenz des Lysinexporters aus C. glutamicum unter Zuhilfenahme des Programmpakets HUSAR (Release 3.0) des Deutschen Krebsforschungszentrums (Heidelberg) mit abgeleiteten Protein-Sequenzen aller dort deponierten DNA-Sequenzen verglichen. Zu einer einzigen Sequenz bisher unbekannter Funktion aus E. coli ergab sich eine hohe Homologie von 39,3 % identischen Aminosäuren, und 64,9 % ähnlichen Aminosäuren. Der Vergleich ist in Figur 2 gezeigt. Das bislang nicht charakterisierte offene Leseraster aus E. coli ist über dieses Verfahren damit als ein Aminosäureexportgen identifiziert.

### e) Gesteigerter Export intrazellulär akkumulierten L-Lysins

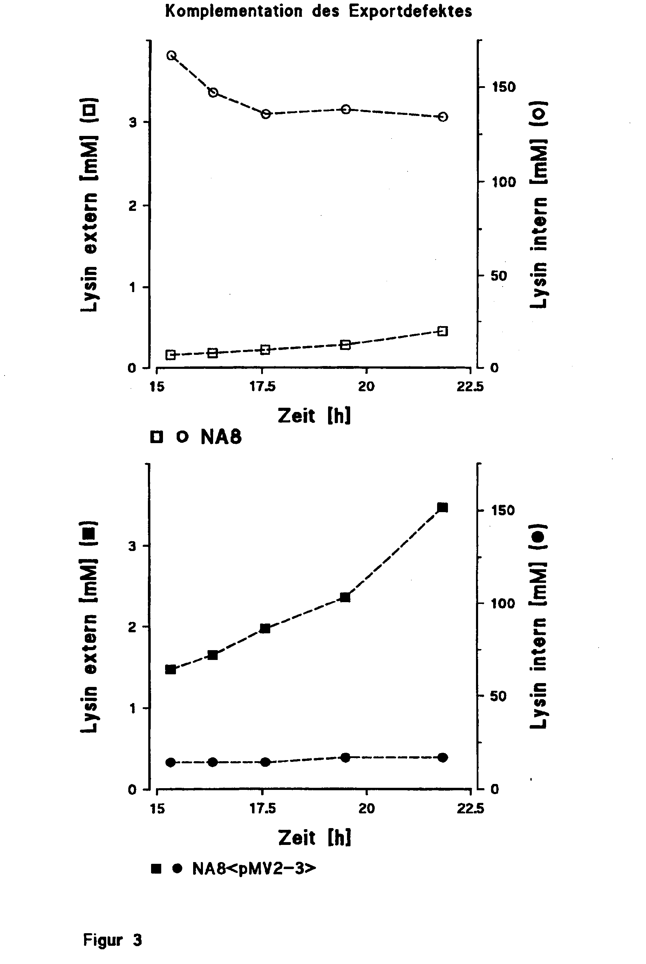

Der Stamm C. glutamicum NA8 (J Bacteriol (1995) 177: 4021-4027) wurde mit Plasmid pMV2-3 transformiert, und die L-Lysinausscheidung der Stämme verglichen. Dazu wurden NA8 und NA8pMV2-3 in Komplexmedium wie bei Vrljic et al. (J Bacteriol (1995) 177: 4021-4027) beschrieben angezogen, und das Fermentationsmedium CGXII (J Bacteriol (1993) 175: 5595-5603) jeweils getrennt beimpft. Das Medium enthielt zusätzlich 5 mM L-Methionin, um die intrazelluläre L-Lysinbiosynthese zu induzieren. Nach Kultivierung für 24 Stunden bei 30 °C auf dem Rotationsschüttler bei 140 Upm wurde zellinterne und externe L-Lysinbestimmungen durchgeführt. Zur zellinternen Bestimmung wurden Silikonölzentrifugationen durchgeführt (Methods Enzymology LV (1979) 547-567); die Bestimmung der Aminosäuren erfolgte mittels Hochdruckflüssigchromatografie (J Chromat (1983) 266: 471-482). Diese Bestimmungen wurden zu verschiedenen Zeiten, wie in Figur 3 angegeben, durchgeführt. Entsprechend dem benutzten Verfahren wird das angestaute zellinterne L-Lysin also durch pMV2-3 vermehrt ausgeschieden und akkumuliert. Entsprechend ist erwartungsgemäß auch das zellintern vorhandene L-Lysins stark reduziert. Somit stellt die Nutzung des entdeckten und beschriebenen Exporters ein Verfahren dar, um die L-Lysinbildung entscheidend zu verbessern.

### f) Gesteigerte Akkumulation von L-Lysin durch lysE oder lysEG

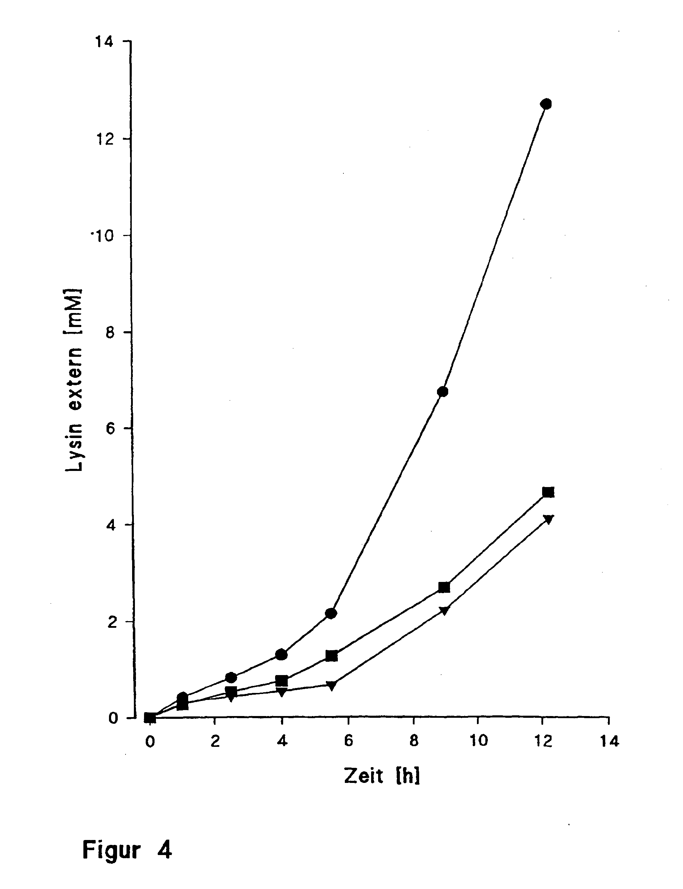

Vom Subclon pMV2-3, der das sequenzierte 2374 bp BamHI-Fragment in pJC1 enthält (siehe Figur 1), wurde entsprechend der Sequenzinformation das lysE tragende 1173 bp PvuII-HindII Fragment in pZ1 (Appl Env Microbiol (1989) 55: 684-688) ligiert, und so das Plasmid plysE erhalten. Dieses Plasmid, sowie das lysElysG tragende Plasmid pMV2-3 wurde durch Elektroporation in C. glutamicum Stamm d eingeführt, indem chromosomale Bereiche deletiert sind. Die erhaltenen Stämme C. glutamicum d pMV2-3, C. glutamicum d plysE, C. glutamicum pJC1 wurden wie unter e) beschrieben zunächst auf Komplexmedium vorgezogen, dann in Produktionsminimalmedium CGXII zusammen mit 4% Glukose und 5 mM L-Methionin kultiviert, und Proben zur Bestimmung des akkumulierten L-Lysins entnommen. Wie aus Figur 4 ersichtlich, wird durch lysElysG eine Steigerung der Lysinakkumulation gegenüber der Kontrolle erreicht. Die plysE wird durch dieses Verfahren eine außerordentlich gesteigerte Akkumulation von 4,8 auf 13,2 mM L-lysin erreicht.

### Legenden der Tabellen und Figuren:

Tabelle 1: Die Aminosäuresequenz des Lysinexporter-Regulators aus Corynebacterium glutamicum, mit dem für DNA-bindende Proteine typischen Helix-Turn-Helix Motif.

Tabelle 2 (drei Seiten): Die Nukleotidsequenz des für den Lysinexporter und Lysinexport-Regulators codierenden Bereichs aus C. glutamicum.

Tabelle 3: Die Aminosäuresequenz des Lysinexporters aus Corynebacterium glutamicum, mit den identifizierten transmembranen Helices TMH1 bis TMH6.

Figur 1: Die durch die Klonierung erhaltenen DNA-Fragmente in pMV6-3 und pMV8-5-24, die die Lysinsekretion bewirken, sowie der aus pMV6-3 hergestellte Subklon pMV2-3, der ebenfalls die Lysinsekretion bewirkt und sequenziert wurde.B, BamHI; Sm, SmaI; Sc, SacI; S1, SalI;H, HindII; X, XhoI.

Figur 2: Vergleich der abgeleiteten Aminosäuresequenz von LysE aus C. glutamicum (oben), mit einem Genprodukt bislang unbekannter Funktion aus Escherichi coli (unten), das dadurch als Exportcarrier identifiziert ist.

Figur 3: Gesteigerter Lysinexport durch pMV2-3 mit C. glutamicum NA8. Oben, die Kontrolle mit geringer Ausscheidung und zellinternem Anstau von Lysin bis etwa 150 mM. Unten die durch pMV2-3 bewirkte hohe Ausscheidung mit zellinternem nur geringem Anstau von etwa 30 mM.

Figur 4: Die Steigerung der Lysinakkumulation in C. glutamicum durch lysElysG (pMV2-3) (mittlere Kurve), und die durch lysE (plysE) bedingte Akkumulation (obere Kurve).

**Tabelle 1**

| | |
|---|---|
| 1 | |
| 51 | TQPAKATEAG EVLVQAARKM VLLQAETKAQ LSGRLAEIPL TIAINADSLS |
| 101 | TWFPPVFNEV ASWGGATLTL RLEDEAHTLS LLRRGDVLGA VTREANPVAG |
| 151 | CEVVELGTMR HLAIATPSLR DAYMVDGKLD WAAMPVLRFG PKDVLQDRDL |
| 201 | DGRVDGPVGR RRVSIVPSAE GFGEAIRRGL GWGLLPETQA APMLKAGEVI |
| 251 | LLDEIPIDTP MYWQRWRLES RSLARLTDAV VDAAIEGLRP |

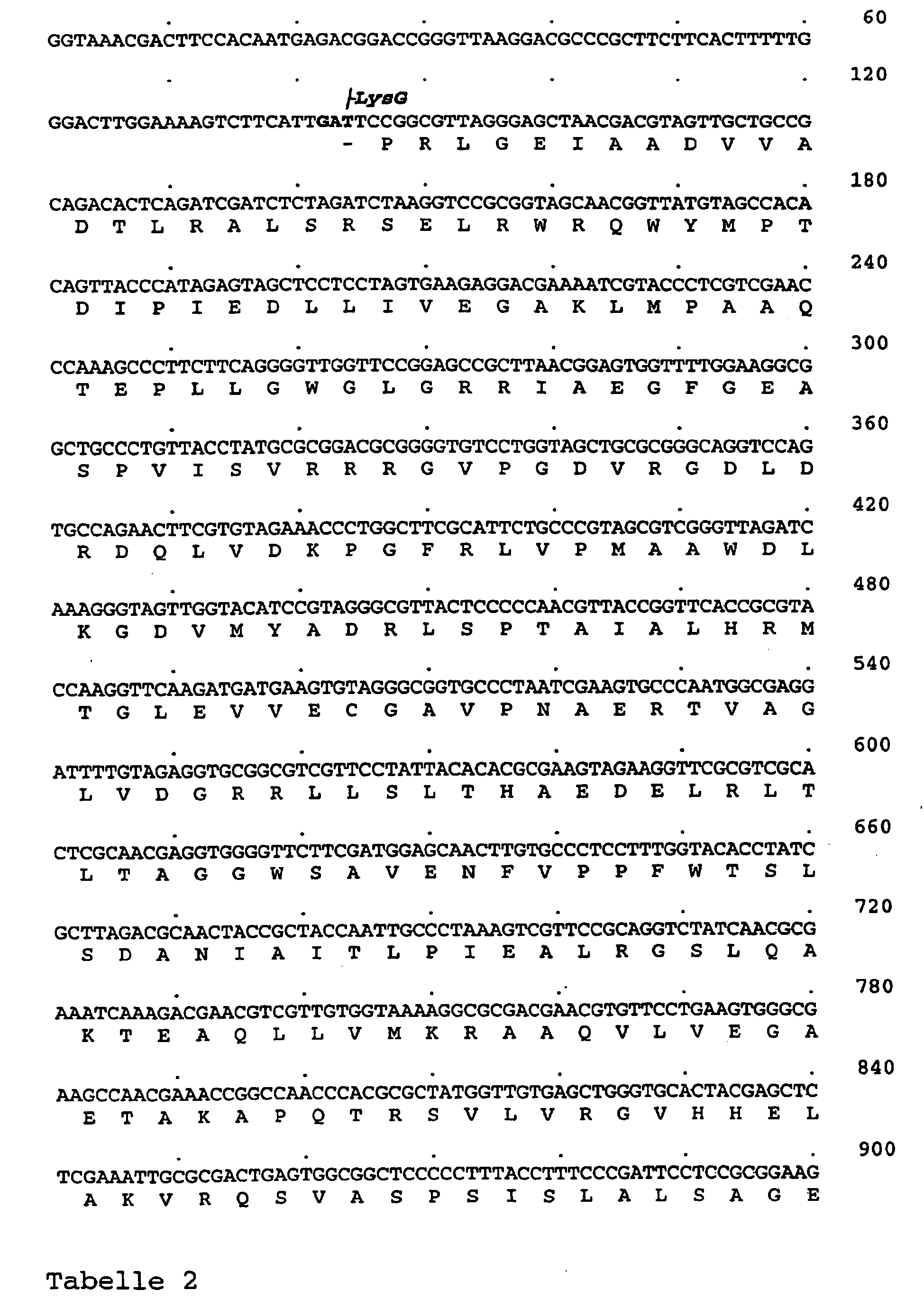

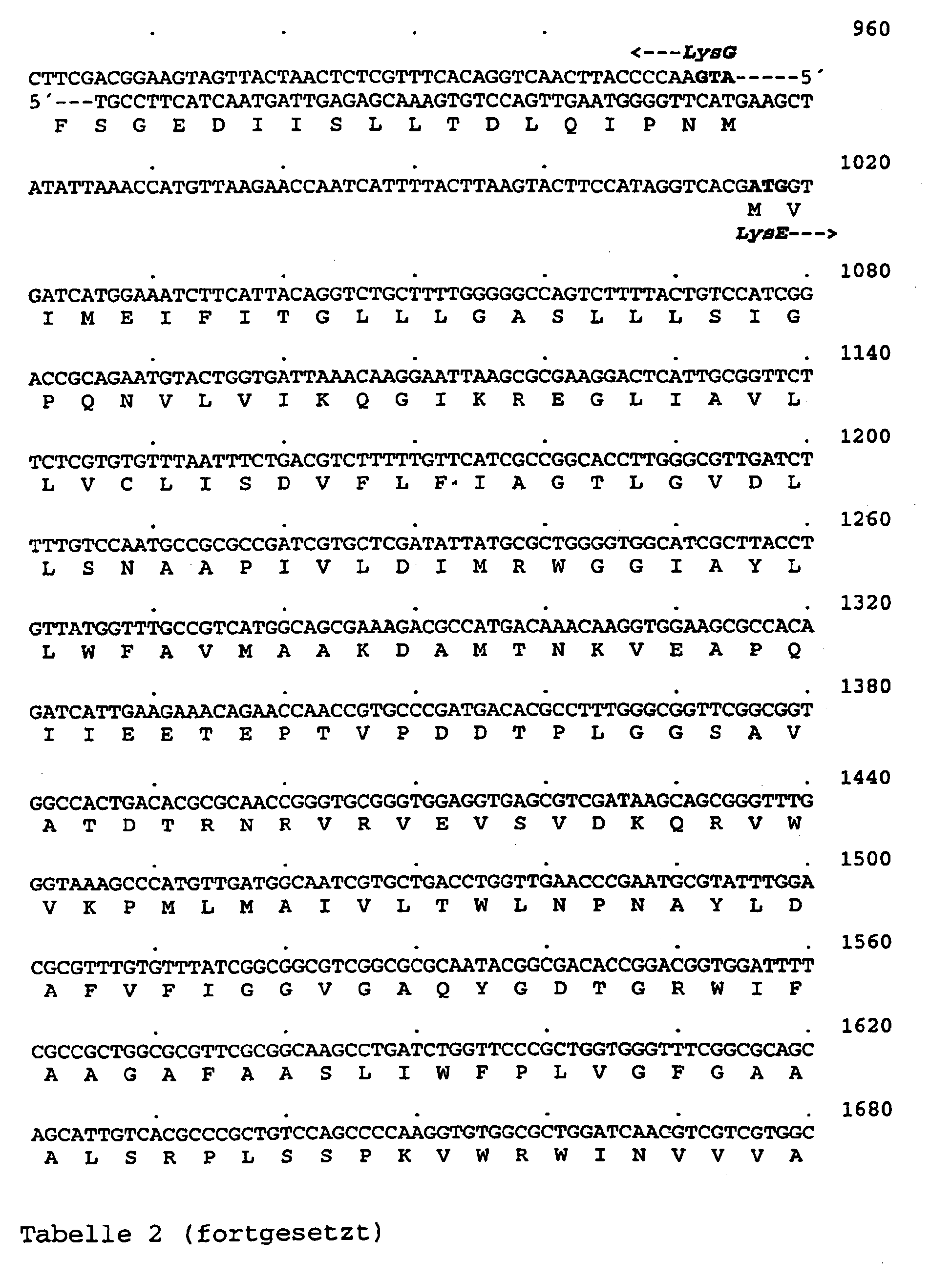

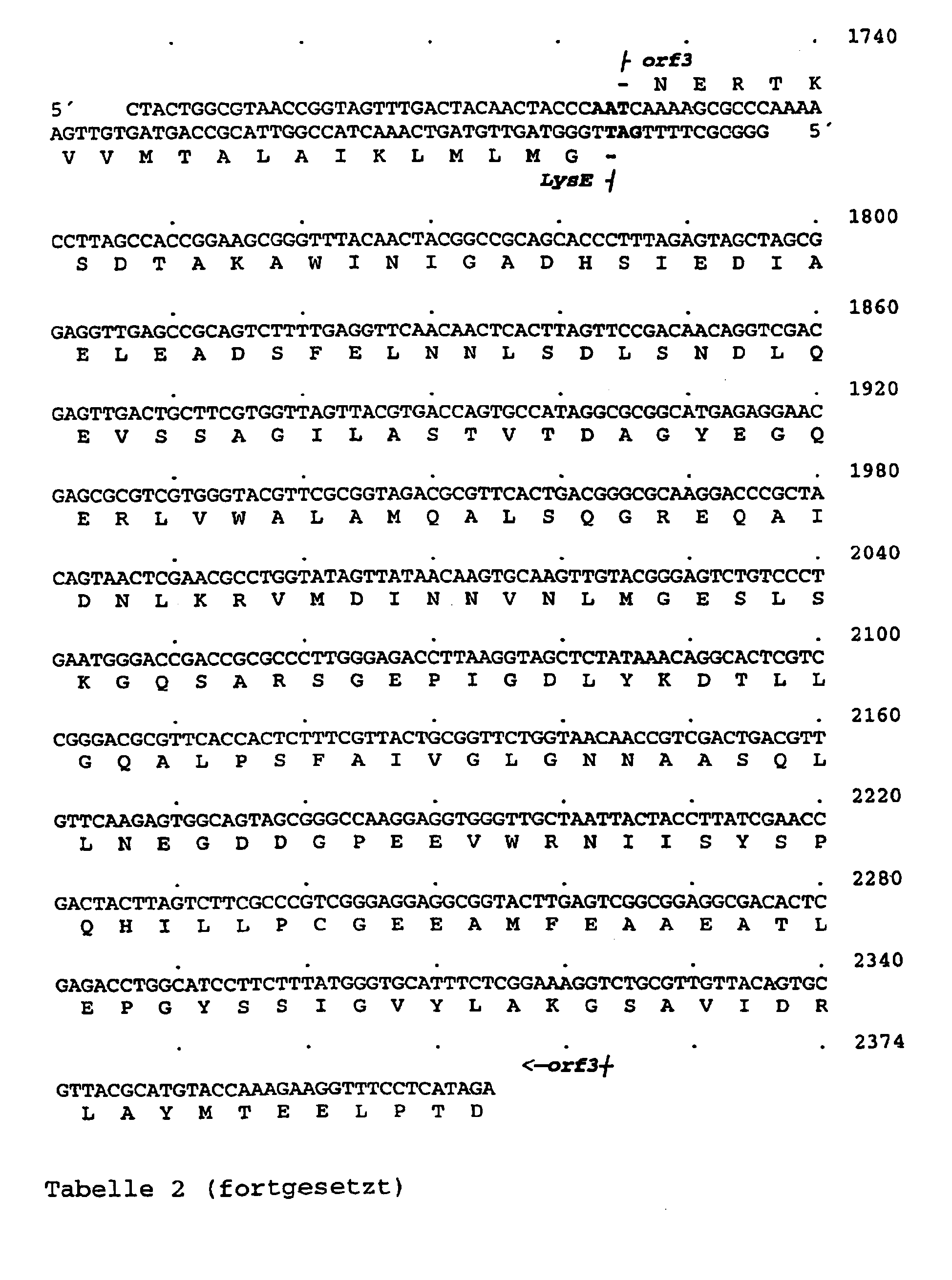

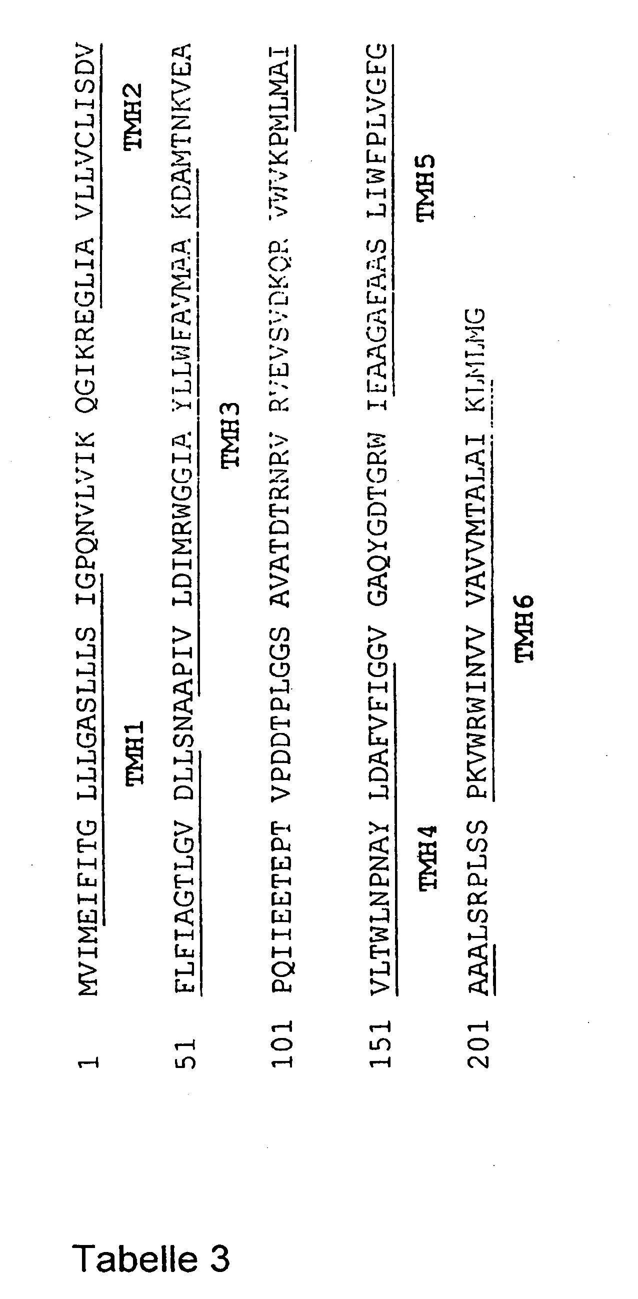

## Patentansprüche

1. Verfahren zur mikrobiellen Herstellung von Aminosäuren, bei dem die für die entsprechende Aminosäure spezifische Exportcarrier-Aktivität von LysE mit einer in SEQ ID No. 2 angegebenen Aminosäuresequenz und/oder die für die entsprechende Aminosäure spezifische Exportgen-Expression von lysE mit der Nukleotidsequeriz von Nukleotid 1016 bis 1726 gemäß SEQ ID No. 1 eines die entsprechende Aminosäure produzierenden Mikroorganismus erhöht wird.

2. Verfahren nach Anspruch 1,
**dadurch gekennzeichnet,**
**daß** die Genexpression des Exportcarriers lysE durch Erhöhen der Genkopienzahl erhöht wird.

3. Verfahren nach Anspruch 2,
**dadurch gekennzeichnet,**
**daß** zur Erhöhung der Genkopienzahl das Exportgen lysE in ein Genkonstrukt eingebaut wird.

4. Verfahren nach Anspruch 3,
**dadurchgekennzeichnet**,
daß das Exportgen lysE in ein Genkonstrukt eingebaut wird, das dem Exportgen zugeordnete regulatorische Gensequenzen enthält.

5. Verfahren nach Anspruch 4,
**dadurch gekennzeichnet,**
**daß** die regulatorische Gensequenz lysG mit einer Aminosäuresequenz gemäß SEQ ID No. 5 eine der regulatorischen Gensequenzen ist.

6. Verfahren nach Anspruch 5,
**dadurch gekennzeichnet,**
**daß** die regulatorische Gensequenz lysG eine Nukleotidsequenz von Nukleotid 1421 bis 2293 gemäß SEQ ID No. 3 aufweist.

7. Verfahren nach einem der Ansprüche 2 bis 6,
**dadurch gekennzeichnet,**
**daß** ein die entsprechende Aminosäure produzierender Mikroorganismus mit dem das Exportgen lysE enthaltende Genkonstrukt transformiert wird.

8. Verfahren nach Anspruch 7,
**dadurch gekennzeichnet,**
**daß** ein Mikroorganismus der Gattung Corynebacterium mit dem das Exportgen lysE enthaltende Genkonstrukt transformiert wird.

9. Verfahren nach Anspruch 7 oder 8,
**dadurch gekennzeichnet,**
**daß** für die Transformation ein Mikroorganismus eingesetzt wird, in dem die an der Synthese der entsprechenden Aminosäure beteiligten Enzyme dereguliert sind.

10. Verfahren nach einem der Ansprüche 7 bis 9,
**dadurch gekennzeichnet,**
**daß** für die Transformation ein Mikroorganismus eingesetzt wird, der einen erhöhten Anteil an Zentralstoffwechselmetaboliten enthält.

11. Verfahren nach einem der Ansprüche 2 bis 10,
**dadurch gekennzeichnet,**
**daß** das Exportgen lys E aus einem Mikroorganismen-Stamm der Gattung Corynebacterium isoliert wird.

12. Verfahren nach einem der vorhergehenden Ansprüche,
**dadurch gekennzeichnet,**
**daß** die lysE Exportgen-Expression durch Verstärkung der Transkriptionssignale erhöht wird.

13. Verfahren nach einem der vorhergehenden Ansprüche zur Herstellung von L-Lysin.

14. Exportgen lysE kodierend für einen Aminosäure-Exportcarrier LysE mit einer für die in SEQ ID No. 2 angegebenen Aminosäuresequenz.

15. Exportgen nach Anspruch 14 mit der Nukleotidsequenz von Nukleotid 1016 bis 1726 gemäß SEQ ID No. 1.

16. Exportgen nach einem der Ansprüche 14 oder 15, das regulatorischen Gensequenzen umfasst.

17. Exportgen nach Anspruch 16,
**dadurch gekennzeichnet,**
**daß** die regulatorische Gensequenz lysG eine für die in SEQ ID No. 5 angegebene Aminosäuresequenz aufweist.

18. Exportgen nach Anspruch 17,
**dadurch gekennzeichnet,**
**daß** die regulatorische Gensequenz lysG eine Nukleotidsequenz von Nukleotid 1421 bis 2293 gemäß SEQ ID No. 3 aufweist.

19. Zur Regulation eines für einen Aminosäure-Exportcarrier kodierenden Exportgens lysE mit einer Nukleotidsequenz gemäß SEQ ID No. 1 geeignetes Regulatorgen lysG mit einer für die in SEQ ID No. 5 angegebene Aminosäuresequenz.

20. Regulatorgen nach Anspruch 19 mit der Nukleotidsequenz von Nukleotid 1421 bis 2293 gemäß SEQ ID No. 3.

21. Genkonstrukt, enthaltend ein Exportgen nach einem der Ansprüche 14 bis 16.

22. Genkonstrukt nach Anspruch 21, zusätzlich enthaltend eine regulatorische Gensequenz nach Anspruch 19 oder 20.

23. Vektor, enthaltend ein Exportgen nach einem der Ansprüche 14 bis 16 oder ein Genkonstrukt nach Anspruch 21.

24. Vektor nach Anspruch 23, zusätzlich enthaltend eine regulatorische Gensequenz nach Anspruch 19 oder 20 oder ein Genkonstrukt nach Anspruch 22.

25. Transformierte Zelle, enthaltend in replizierbarer Form ein Exportgen nach einem der Ansprüche 14 bis 16 oder ein Genkonstrukt nach Anspruch 21.

26. Transformierte Zelle nach Anspruch 25, enthaltend einen Vektor nach Anspruch 23.

27. Transformierte Zelle nach Anspruch 25 oder 26
**dadurch gekennzeichnet,**
**daß** sie der Gattung Corynebacterium angehört.

28. Transformierte Zelle nach einem der Ansprüche 25 bis 27,
**dadurch gekennzeichnet,**
**daß** in dieser die an der Synthese beteiligten Enzyme der Aminosäure, die mittels des Exportcarriers, für das das in die transformierte Zelle übertragene Exportgen lysE kodiert, aus der Zelle ausgeschleust wird, dereguliert sind.

29. Transformierte Zelle nach einem der Ansprüche 25 bis 28,
**dadurch gekennzeichnet,**
**daß** sie einen erhöhten Anteil an Zentralstoffwechselmetaboliten enthält.

30. Transformierte Zelle nach einem der Ansprüche 25 bis 29, zusätzlich enthaltend in replizierbarer Form eine regulatorische Gensequenz lysG nach Anspruch 19 oder 20 oder ein Genkonstrukt nach Anspruch 22.

31. Transformierte Zelle nach Anspruch 30, enthaltend einen Vektor nach Anspruch 24.

32. Aminosäure-Exportcarrier LysE mit 6 transmembranen Helices, kodiert durch ein Exportgen lysE mit der in SEQ ID No. 2 angegebenen Aminosäuresequenz.

33. Verwendung des Exportgens lysE mit einer Nukleotidsequenz gemäß SEQ ID No. 1 zur Steigerung der Aminosäureproduktion von Mikroorganismen.

34. Verwendung nach Anspruch 33,
**dadurch gekennzeichnet**
**daß** das Exportgen lysE, dessen Genexpression des Exportcarriers lysE durch
Erhöhen der Genkopienzahl und/oder durch Verwendung regulatorischer Faktoren, die die Exportgen-Expression positiv beeinflussen erhöht wird, eingesetzt wird.

35. Verwendung nach Anspruch 33 oder 34,
**dadurch gekennzeichnet,**
**daß** der Aminosäure-produzierende Mikroorganismus mit einem Genkonstrukt, das das Exportgen lysE enthält, transformiert wird.

36. Verwendung nach Anspruch 35,
**dadurch gekennzeichnet,**
**daß** das Genkonstrukt zusätzlich regulatorische Gensequenzen trägt.

37. Verwendung nach einem der Ansprüche 33 bis 36,
**dadurch gekennzeichnet,**
**daß** das Exportgen lysE aus Corynebacterium eingesetzt wird.

38. Verwendung nach einem der Ansprüche 33 bis 37,
**dadurch gekennzeichnet,**
**daß** als Aminosäure-produzierender Mikroorganismus Corynebacterium eingesetzt wird.

## Claims

1. Process for microbial production of amino acids, in which the LysE export carrier activity specific for the respective amino acid, with LysE having an amino acid sequence indicated in SEQ ID No. 2, and/or lysE export gene expression specific for the respective amino acid, with lysE having the nucleotide sequence of nucleotides 1016 to 1726 as set forth in SEQ ID No. 1, of a microorganism producing the respective amino acid is increased.

2. Process according to Claim 1, **characterized in that** gene expression of the export carrier lysE is increased by increasing the number of gene copies.

3. Process according to Claim 2, **characterized in that** the number of gene copies is increased by incorporating the export gene lysE into a gene construct.

4. Process according to Claim 3, **characterized in that** the export gene lysE is incorporated into a gene construct comprising regulatory gene sequences linked to the export gene.

5. Process according to Claim 4, **characterized in that** one of the regulatory gene sequences is the lysG regulatory gene sequence having an amino acid sequence as set forth in SEQ ID No. 5.

6. Process according to Claim 5, **characterized in that** the lysG regulatory gene sequence has a nucleotide sequence of nucleotides 1421 to 2293 as set forth in SEQ ID No. 3.

7. Process according to any of Claims 2 to 6,
**characterized in that** a microorganism producing the respective amino acid is transformed with the gene construct comprising the lysE export gene.

8. Process according to Claim 7, **characterized in that** a microorganism of the genus Corynebacterium is transformed with the gene construct comprising the lysE export gene.

9. Process according to Claim 7 or 8, **characterized in that** a microorganism is employed for transformation, in which the enzymes involved in the synthesis of the respective amino acid are deregulated.

10. Process according to any of Claims 7 to 9,
**characterized in that** a microorganism is employed for transformation, in which an increased proportion of metabolites of the central metabolism is present.

11. Process according to any of Claims 2 to 10,
**characterized in that** the lysE export gene is isolated from a microorganism strain of the genus Corynebacterium.

12. Process according to any of the preceding claims, **characterized in that** lysE export gene expression is increased by enhancing the transcription signals.

13. Process according to any of the preceding claims for producing L-lysine.

14. Export gene lysE coding for a LysE amino acid export carrier having an amino acid sequence indicated in SEQ ID No. 2.

15. Export gene according to Claim 14, having the nucleotide sequence of nucleotides 1016 to 1726 as set forth in SEQ ID No. 1.

16. Export gene according to either of Claims 14 and 15, which comprises regulatory gene sequences.

17. Export gene according to Claim 16, **characterized in that** the lysG regulatory gene sequence has an amino acid sequence indicated in SEQ ID No. 5.

18. Export gene according to Claim 17, **characterized in that** the lysG regulatory gene sequence has a nucleotide sequence of nucleotides 1421 to 2293 as set forth in SEQ ID No. 3.

19. Regulatory gene lysG useful for regulating a lysE export gene coding for an amino acid export carrier and having a nucleotide sequence as set forth in SEQ ID No. 1, which regulatory gene has an amino acid sequence indicated in SEQ ID No. 5.

20. Regulatory gene according to Claim 19 having the nucleotide sequence of nucleotides 1421 to 2293 as set forth in SEQ ID No. 3.

21. Gene construct comprising an export gene according to any of Claims 14 to 16.

22. Gene construct according to Claim 21, additionally comprising a regulatory gene sequence according to Claim 19 or 20.

23. Vector comprising an export gene according to any of Claims 14 to 16 or a gene construct according to Claim 21.

24. Vector according to Claim 23, additionally comprising a regulatory gene sequence according to Claim 19 or 20 or a gene construct according to Claim 22.

25. Transformed cell comprising, in a replicable form, an export gene according to any of Claims 14 to 16 or a gene construct according to Claim 21.

26. Transformed cell according to Claim 25, comprising a vector according to Claim 23.

27. Transformed cell according to Claim 25 or 26,
**characterized in that** it belongs to the genus Corynebacterium.

28. Transformed cell according to any of Claims 25 to 27, **characterized in that** the enzymes therein, which are involved in the synthesis of the amino acid which is exported from the cell by means of the export carrier encoded by the lysE export gene transferred to the transformed cell, are deregulated.

29. Transformed cell according to any of Claims 25 to 28, **characterized in that** it comprises an increased proportion of metabolites of the central metabolism.

30. Transformed cell according to any of Claims 25 to 29, additionally comprising, in a replicable form, a lysG regulatory gene sequence according to Claim 19 or 20 or a gene construct according to Claim 22.

31. Transformed cell according to Claim 30, comprising a vector according to Claim 24.

32. Amino acid export carrier LysE having 6 transmembrane helices, encoded by a lysE export gene and having the amino acid sequence indicated in SEQ ID No. 2.

33. Use of the lysE export gene having a nucleotide sequence as set forth in SEQ ID No. 1 for increasing amino acid production by microorganisms.

34. Use according to Claim 33, **characterized in that** the lysE export gene whose gene expression of the lysE export carrier is increased by increasing the number of gene copies and/or by using regulatory factors which have a beneficial influence on export gene expression is used.

35. Use according to Claim 33 or 34, **characterized in that** the amino acid-producing microorganism is transformed with a gene construct comprising the lysE export gene.

36. Use according to Claim 35, **characterized in that** the gene construct additionally carries regulatory gene sequences.

37. Use according to any of Claims 33 to 36,
**characterized in that** the lysE export gene of Corynebacterium is employed.

38. Use according to any of Claims 33 to 37,
**characterized in that** the amino acid-producing microorganism employed is Corynebacterium.

## Revendications

1. Procédé de préparation microbienne d'acides aminés, dans lequel on augmente l'activité spécifique de l'acide aminé correspondant, du support d'exportation LysE ayant une séquence d'acides aminés indiquée dans SEQ ID N°: 2, et/ou l'expression spécifique de l'acide aminé correspondant, du gène d'exportation LysE ayant la séquence nucléotidique allant du nucléotide 1016 au nucléotide 1726 selon SEQ ID N°: 1 d'un microorganisme produisant l'acide aminé correspondant.

2. Procédé selon la revendication 1, **caractérisé en ce que** l'expression génique du support d'exportation LysE est augmentée par une augmentation du nombre de copies de gènes.

3. Procédé selon la revendication 2, **caractérisé en ce que**, pour augmenter le nombre de copies de gènes, on incorpore le gène d'exportation LysE dans une construction génique.

4. Procédé selon la revendication 3, **caractérisé en ce que** le gène d'exportation LysE est incorporé dans une construction génique qui contient les séquences géniques régulatrices affectées au gène d'exportation.

5. Procédé selon la revendication 4, **caractérisé en ce que** la séquence génique régulatrice LysG, comportant une séquence d'acides aminés selon SEQ ID N°: 5, est l'une des séquences géniques régulatrices.

6. Procédé selon la revendication 5, **caractérisé en ce que** la séquence génique régulatrice LysG comporte une séquence nucléotidique allant du nucléotide 1421 au nucléotide 2293 selon SEQ ID N°: 3.

7. Procédé selon l'une des revendications 2 à 6, **caractérisé en ce qu'**un microorganisme produisant l'acide aminé correspondant est transformé avec la construction génique contenant le gène d'exportation LysE.

8. Procédé selon la revendication 7, **caractérisé en ce qu'**un microorganisme de l'espèce Corynebacterium est transformé avec la construction génique contenant le gène d'exportation LysE.

9. Procédé selon la revendication 7 ou 8,
**caractérisé en ce qu'**on utilise pour la transformation un microorganisme dans lequel les enzymes participant à la synthèse de l'acide aminé correspondant sont dérégulées.

10. Procédé selon l'une des revendications 7 à 9, **caractérisé en ce qu'**on utilise pour la transformation un microorganisme qui contient une proportion accrue de métabolites du métabolisme central.

11. Procédé selon l'une des revendications 2 à 10, **caractérisé en ce que** le gène d'exportation LysE est isolé d'une souche de microorganisme de l'espèce Corynebacterium.

12. Procédé selon l'une des revendications précédentes, **caractérisé en ce que** l'expression du gène d'exportation LysE est augmentée par un renforcement des signaux de transcription.

13. Procédé selon l'une des revendications précédentes, pour préparer la L-lysine.

14. Gène d'exportation LysE, codant pour un support d'exportation d'acides aminés LysE présentant une séquence d'acides aminés telle qu'indiquée dans SEQ ID N°: 2.

15. Gène d'exportation selon la revendication 14, comportant la séquence nucléotidique allant du nucléotide 1016 au nucléotide 1726 selon SEQ ID N°: 1.

16. Gène d'exportation selon l'une des revendications 14 ou 15, qui comprend des séquences géniques régulatrices.

17. Gène d'exportation selon la revendication 16, **caractérisé en ce que** la séquence génique régulatrice LysE comprend une séquence d'acides aminés indiquée dans SEQ ID N°: 5.

18. Gène d'exportation selon la revendication 17, **caractérisé en ce que** la séquence génique régulatrice LysE comprend une séquence nucléotidique allant du nucléotide 1421 au nucléotide 2293 selon SEQ ID N°: 3.

19. Gène régulateur convenant à la régulation d'un gène d'exportation LysE codant pour un support d'exportation d'acide aminé, ayant une séquence nucléotidique selon SEQ ID N°: 1, et comportant une séquence d'acides aminés telle qu'indiquée dans SEQ ID N°: 5.

20. Gène régulateur selon la revendication 19, comportant la séquence nucléotidique allant du nucléotide 1421 au nucléotide 2293 selon SEQ ID N°: 3.

21. Construction génique contenant un gène d'exportation selon l'une des revendications 14 à 16.

22. Construction génique selon la revendication 21, contenant en outre une séquence génique régulatrice selon la revendication 19 ou 20.

23. Vecteur contenant un gène d'exportation selon l'une des revendications 14 à 16 ou une construction génique selon la revendication 21.

24. Vecteur selon la revendication 23, contenant en outre une séquence génique régulatrice selon la revendication 19 ou 20 ou une construction génique selon la revendication 22.

25. Cellule transformée contenant sous une forme réplicable un gène d'exportation selon l'une des revendications 14 à 16 ou une construction génique selon la revendication 21.

26. Cellule transformée selon la revendication 25, contenant un vecteur selon la revendication 23.

27. Cellule transformée selon la revendication 25 ou 26, **caractérisée en ce qu'**elle appartient à l'espèce Corynebacterium.

28. Cellule transformée selon l'une des revendications 25 à 27, **caractérisée en ce que**, dans cette dernière, les enzymes, qui participent à la synthèse, de l'acide aminé, qui est évacué de la cellule au moyen du support d'exportation pour lequel le gène d'exportation LysE transféré dans la cellule transformée code, subissent une dérégulation.

29. Cellule transformée selon l'une des revendications 25 à 28, **caractérisée en ce qu'**elle contient une proportion accrue de métabolites du métabolisme central.

30. Cellule transformée selon l'une des revendications 25 à 29, contenant en outre sous une forme réplicable une séquence génique régulatrice LysG selon la revendication 19 ou 20 ou une construction génique selon la revendication 22.

31. Cellule transformée selon la revendication 30, contenant un vecteur selon la revendication 24.

32. Support d'exportation d'acide aminé LysE comportant 6 hélices transmembranaires, codé par un gène d'exportation LysE ayant la séquence d'acides aminés indiquée dans SEQ ID N°: 2.

33. Utilisation du gène d'exportation LysE, comportant une séquence nucléotidique selon SEQ ID N°: 1, pour augmenter la production d'acides aminés par des microorganismes.

34. Utilisation selon la revendication 33,
**caractérisée en ce qu'**on utilise le gène d'exportation LysE, dont l'expression génique du support d'expression LysE est augmentée par une augmentation du nombre de copies de gène et/ou par utilisation de facteurs régulateurs qui influent d'une manière positive sur l'expression du gène d'exportation.

35. Utilisation selon la revendication 33 ou 34, **caractérisée en ce que** le microorganisme produisant l'acide aminé est transformé à l'aide d'une construction génique contenant le gène d'exportation LysE.

36. Utilisation selon la revendication 35,
**caractérisée en ce que** la construction génique porte en outre des séquences géniques régulatrices.

37. Utilisation selon l'une des revendications 33 à 36, **caractérisée en ce qu'**on utilise le gène d'exportation LysE de Corynebacterium.

38. Utilisation selon l'une des revendications 33 à 37, **caractérisée en ce qu'**on utilise Corynebacterium en tant que microorganisme produisant des acides aminés.
